# EUROPEAN PATENT APPLICATION

(11) **EP 4 692 315 A1**
(43) Date of publication of application: **11.02.2026**
(21) Application number: 24785353.4
(22) Date of filing: 05.04.2024
(51) Int. Cl.: C12N 5/00

(54) **WATER-SOLUBLE DECELLULARIZED SUBSTRATE BASED ON SUPERCRITICAL CARBON DIOXIDE PROCESS**

(30) Priority: 06.04.2023 KR 20230045449
(71) Applicant: Medifab Co., Ltd., Seoul 08594 (KR)
(72) Inventor: KIM, Su Hee, Seoul 07916 (KR); CHA, Mi Sun, Seoul 08102 (KR); KANG, Jae Jun, Seoul 08586 (KR)
(74) Representative: Morabito, Sara
(86) International application number: PCT/KR2024/004578
(87) International publication number: WO 2024/210656

(57) **Abstract**

According to a method of an aspect, the content and biological activity of bioactive substances in the dECM may be maintained during a dissolution process of the dECM through a supercritical carbon dioxide process, and low osmotic pressure may be maintained.

Therefore, a water-soluble dECM composition, prepared according to the method of an aspect, and a dECM material including the same have low biotoxicity, and excellent tissue regeneration ability, and may be efficiently complexed with other substances, and thus may be effectively used as a cosmetic or medical material.

## Description

### Technical Field

The present disclosure relates to a method of preparing a water-soluble decellularized extracellular matrix (dECM) having improved biological activity.

### Background Art

The extracellular matrix (ECM) is a non-cellular structure of a tissue or organ, composed of various polymers secreted from cells. The ECM may be used as a natural scaffold material that is structurally intact and usable for biological purposes. Their specific composition and function may appear differently depending on the form of the particular tissue of origin.

The decellularization process is a process of separating ECM components from biological tissue through chemical treatment. Through the decellularization process, cellular components and other immunogenic components are removed from the biological tissue, while bioactive substances involved in realizing the environment and functions of biological tissue, such as ECM and certain growth factor proteins, may be preserved. Accordingly, such a dECM including various preserved bioactive substances, for example collagen, glycosaminoglycan, and various cytokines, promotes tissue regeneration and provides a more natural biomimetic microenvironment for cells to grow and differentiate. Such a dECM may be used alone or in a composite state together with other materials for purposes such as tissue regeneration.

On the other hand, in the decellularization process or in the process of dissolving the decellularized ECM produced thereby, depending on reaction conditions such as solvent, degrading enzyme, temperature condition, and pH condition, bioactive substances in the dECM may be removed or denatured, resulting in a reduction in biological activity. In addition, conventional methods using surfactants may cause the dECM to contain residual surfactant, which may exhibit toxicity upon in vivo application, and furthermore, depending on the reaction conditions, the osmotic pressure of a composition containing the dECM may increase, and as the osmotic pressure increases, the level of mixing with other substances may decrease, and the efficiency of compounding with other cosmetic or medical materials or components may decrease.

Accordingly, in order to solve the above problems, the present inventors have developed a technology for preparing a dECM composition that is easy to compound with other substances while maintaining the content of bioactive substances and the biological activity in the dECM and at the same time maintaining low osmotic pressure.

### Disclosure

### Technical Problem

An aspect provides a method of dissolving a decellularized extracellular matrix (dECM), comprising treating a solution comprising a dECM powder with supercritical carbon dioxide, wherein the treating with the supercritical carbon dioxide is performed at 31 to 60 °C.

Another aspect provides a method of preparing a water-soluble dECM composition, comprising treating a solution comprising a dECM powder with supercritical carbon dioxide, wherein the treating with the supercritical carbon dioxide is performed at 31 to 60 °C.

Another aspect provides a water-soluble dECM composition prepared by the method of preparing a water-soluble dECM composition.

Other objects and advantages of the present application will be made clearer by the following detailed description together with the accompanying claims and drawings. Matters not described in the present specification may be sufficiently recognized and inferred by those skilled in the art to which the present disclosure pertains or to a similar technical field, and thus description thereof is omitted.

### Technical Solution

Throughout the present specification, when a part is described as "comprising" a component, it means that, unless specifically stated otherwise, it does not exclude other components, but may further include other components. In addition, unless the context clearly specifies a particular order, each process may be carried out in an order different from that stated. That is, each process may be carried out in the same order as stated, substantially simultaneously, or in the reverse order.

An aspect provides a method of dissolving a dECM, including
treating a solution comprising a dECM powder with supercritical carbon dioxide, wherein the treating with the supercritical carbon dioxide is performed at 31 to 60 °C.

As used herein, the term "decellularized substrate" may be used interchangeably with "decellularized tissue," "decellularized extracellular matrix (dECM)," or "decellularized material." The dECM means that decellularization is performed on tissue or organs of a human or an animal such as a pig or a cow, to remove cellular components other than the extracellular matrix, for example, nucleus, cell membrane, nucleic acids, and the like. On the other hand, "decellularized organ" refers to one in which an entire organ, such as a heart or kidney, is decellularized while maintaining the overall structure of the organ, and since its purpose is to re-implant cells or to use the organ while maintaining the physical structure of the whole organ, water-soluble formulation of the extracellular matrix is impossible. Therefore, it is clearly a concept distinguished from the "dECM" of the present disclosure.

In an embodiment, the dECM may be derived from human, or animal tissue such as skin tissue, adipose tissue, cardiac tissue, corneal tissue, or cartilage tissue of animals such as pigs or cows, but is not limited thereto.

The extracellular matrix (ECM) refers to a complex assembly of biomacromolecules filling the spaces within tissues or the extracellular spaces. The ECM is composed of various types of molecules that are synthesized by cells and secreted and accumulated outside the cells, such as fibrous proteins, complex proteins such as proteoglycans, and cell-adhesive proteins such as fibronectin and laminin. Accordingly, the components of the ECM may vary depending on the type of the originating cells or the degree of differentiation of the cells.

On the other hand, the reaction conditions of the decellularization process, for example, the solvent used, degrading enzymes, temperature conditions, pH conditions, and the like, in practice affect the content of bioactive substances in the dECM, the degree of biological activity thereof, the residue of toxic substances (for example, surfactants), and osmotic pressure.

The term "solution" as used herein may mean a state in which two substances are mixed, for example, a state in which a solute and a solvent are mixed. In an embodiment, the solution including the dECM powder may mean a mixture, suspension or aqueous solution in which the dECM powder is contained in distilled water. The term "solution" as used herein may be used interchangeably with mixture, suspension, hydrate, composition, solubilizate, or aqueous solution.

The term "dissolution" as used herein refers to a phenomenon in which a solute is mixed into a solvent, and may refer to, for example, that the solubility of the solute in the solvent increases compared to a previous state. In an embodiment, the method of dissolving the dECM may mean increasing the solubility of the powder in distilled water in a mixture containing the dECM powder. In an embodiment, the method of dissolving the dECM may increase the solubility of the powder while maintaining the osmotic pressure of the solution lower than in methods of treating with acidic or alkaline solvents. In an embodiment, the method of dissolving the dECM may increase solubility while maintaining the pH of the solution neutral. In an embodiment, the method of dissolving the dECM may increase solubility while maintaining the biological activity of bioactive substances in the dECM, and may increase solubility while maintaining the biocompatibility of the solution.

The powder of the dECM may be a dried powder of the extracellular matrix that has undergone a decellularization process, and may be obtained by drying the extracellular matrix that has undergone a decellularization process and pulverizing it into a powder. For example, it may be obtained by drying a decellularized extracellular matrix that has undergone a decellularization process and then pulverizing it into a powder. The drying method of preparing the extracellular matrix in a dried state may employ methods generally used in the art and is not particularly limited. Non-limiting examples of the drying method include air drying, natural drying, spray drying, freeze-drying, and vacuum drying. These methods may be used alone or in combination of at least two methods. In an embodiment, the dECM dried powder may be a freeze-dried powder of the dECM.

In an embodiment, the method of dissolving the dECM powder may be a method of dissolving the dECM powder in distilled water, and may not additionally include treating with an acidic or alkaline solvent.

The solution comprising the dECM powder may include the powder of the dECM and distilled water. In an embodiment, the powder of the dECM may be contained in distilled water at 0.01 to 5 wt%, for example, 0.05 to 5 wt%, 0.1 to 5 wt%, 0.2 to 5 wt%, 0.3 to 5 wt%, 0.4 to 5 wt%, 0.5 to 5 wt%, 0.6 to 5 wt%, 0.7 to 5 wt%, 0.8 to 5 wt%, 0.9 to 5 wt%, 1.0 to 5 wt%, 0.05 to 4 wt%, 0.1 to 4 wt%, 0.2 to 4 wt%, 0.3 to 4 wt%, 0.4 to 4 wt%, 0.5 to 4 wt%, 0.6 to 4 wt%, 0.7 to 4 wt%, 0.8 to 4 wt%, 0.9 to 4 wt%, 1.0 to 4 wt%, 0.05 to 3 wt%, 0.1 to 3 wt%, 0.2 to 3 wt%, 0.3 to 3 wt%, 0.4 to 3 wt%, 0.5 to 3 wt%, 0.6 to 3 wt%, 0.7 to 3 wt%, 0.8 to 3 wt%, 0.9 to 3 wt%, or 1.0 to 3 wt%, but is not limited thereto.

The treating with the supercritical carbon dioxide may include an injecting supercritical carbon dioxide into a reactor containing the dECM powder and distilled water, which serves to dissolve the dECM powder in the distilled water. The composition containing the dECM dissolved in this way may thereafter be formulated to have desired properties or complexed with other substances, and used as an efficient cosmetic or medical material. The process of placing the dECM powder and distilled water into the reactor and of injecting the supercritical carbon dioxide may be carried out simultaneously or sequentially.

In an embodiment, the treating with the supercritical carbon dioxide may be carried out at a temperature of 31 to 60 °C in the reactor. If the temperature in the reactor falls below this range, there is a concern that the dECM powder may not be sufficiently dissolved, and conversely, if the temperature in the reactor rises above this range, there is a concern that bioactive substances and other effective components in the dECM may be decomposed or otherwise denatured. For example, the treating with the supercritical carbon dioxide may be performed under conditions where the temperature inside the reactor is 31 to 55 °C, 31 to 50 °C, 31 to 45 °C, 31 to 40 °C, 31 to 39 °C, 31 to 38 °C, 31 to 37 °C, 32 to 40 °C, 33 to 40 °C, 34 to 40 °C, 35 to 40 °C, 32 to 39 °C, 33 to 39 °C, 34 to 39 °C, 35 to 39 °C, 32 to 38 °C, 33 to 38 °C, 34 to 38 °C, 35 to 38 °C, 32 to 37 °C, 33 to 37 °C, 34 to 37 °C, or 35 to 37°C, but is not limited thereto.

In an embodiment, the treating with the supercritical carbon dioxide may be carried out in a reactor, and may include injecting carbon dioxide gas into the reactor to maintain a pressure in the reactor at 70 to 400 bar. If the pressure in the reactor falls outside the range and is too low, there is a concern that the dECM powder may not be sufficiently dissolved. For example, it may include injecting carbon dioxide gas into the reactor to maintain the pressure in the reactor at 80 to 400 bar, 90 to 400 bar, 100 to 400 bar, 110 to 400 bar, 120 to 400 bar, 130 to 400 bar, 140 to 400 bar, 150 to 400 bar, 160 to 400 bar, 170 to 400 bar, 180 to 400 bar, 190 to 400 bar, 150 to 390 bar, 150 to 380 bar, 150 to 370 bar, 150 to 360 bar, 150 to 350 bar, 160 to 350 bar, 170 to 350 bar, 180 to 350 bar, 190 to 350 bar, 200 to 350 bar, 210 to 350 bar, 220 to 350 bar, 230 to 350 bar, 240 to 350 bar, 250 to 350 bar, 260 to 350 bar, 270 to 350 bar, 280 to 350 bar, 290 to 350 bar, or 300 to 350 bar, but is not limited thereto.

In an embodiment, the treating with the supercritical carbon dioxide may be performed for 2 to 24 hours, and, for example, may include maintaining the pressure in the reactor for 2 to 24 hours. For example, it may be 3 to 23 hours, 4 to 22 hours, 5 to 21 hours, 6 to 20 hours, 6 to 19 hours, 6 to 18 hours, 6 to 17 hours, 6 to 16 hours, 6 to 15 hours, 6 to 14 hours, 6 to 13 hours, or 6 to 12 hours, but is not limited thereto. For example, the treating with the supercritical carbon dioxide may include maintaining the pressure in the reactor for 6 to 12 hours.

In an embodiment, the method of dissolving the powder of the dECM may not include treating with an acid or alkaline solution.

The method of dissolving the powder of the dECM according to an embodiment includes, unlike conventional dissolution methods, a process of treating with supercritical carbon dioxide performed at a specific temperature, and as an effect therefrom, it does not include a subsequent process of treating with an acid or alkaline solution or a subsequent process of treating with a degrading enzyme, thereby having high efficiency in the manufacturing process and not requiring a separate purification process. Accordingly, the water-soluble dECM composition produced by the method according to an embodiment maintains the content of bioactive substances and the biological activity thereof, may maintain low osmotic pressure, is easy to mix with other components or substances, and exhibits excellent biocompatibility.

The dECM may be prepared by a decellularization method including injecting supercritical carbon dioxide into a reactor containing biological tissue and ethanol to maintain a pressure in the reactor at 70 to 400 bar, wherein the process of injecting the supercritical carbon dioxide into the reactor containing the biological tissue and ethanol may be performed at 31 to 60 °C.

In an embodiment, the biological tissue may be skin tissue, adipose tissue, cardiac tissue, corneal tissue, or cartilage tissue of a human or of an animal such as a pig or cow, but is not limited thereto.

The injecting supercritical carbon dioxide into the reactor containing the biological tissue and ethanol may be such that a process of placing the biological tissue and ethanol into the reactor and a process of injecting the supercritical carbon dioxide are carried out simultaneously or sequentially, and this serves to remove cellular components and other immunogenic components in the biological tissue through high pressure.

In an embodiment, the injecting supercritical carbon dioxide into the reactor containing the biological tissue and ethanol may be performed at 31 to 60 °C. If the temperature in the reactor falls outside this range and is too low, there is a concern that the decellularization process may not sufficiently occur and the immunogenicity of the dECM may not be sufficiently removed, and if the temperature in the reactor is too high, there is a concern that effective components such as bioactive substances in the extracellular matrix may be decomposed or otherwise denatured during the decellularization process. For example, the treating with the supercritical carbon dioxide may be carried out under conditions in which the temperature in the reactor is 31 to 55 °C, 31 to 50 °C, 31 to 45 °C, 31 to 40 °C, 31 to 39 °C, 31 to 38 °C, 31 to 37 °C, 32 to 40 °C, 33 to 40 °C, 34 to 40 °C, 35 to 40 °C, 32 to 39 °C, 33 to 39 °C, 34 to 39 °C, 35 to 39 °C, 32 to 38 °C, 33 to 38 °C, 34 to 38 °C, 35 to 38 °C, 32 to 37 °C, 33 to 37 °C, 34 to 37 °C, or 35 to 37 °C, but is not limited thereto.

In an embodiment, the injecting supercritical carbon dioxide into the reactor containing the biological tissue and ethanol may include injecting carbon dioxide into the reactor to maintain a pressure in the reactor at 70 to 400 bar. If the pressure in the reactor falls outside this range and is too low, there is a concern that the decellularization process may not sufficiently occur and the immunogenicity of the dECM may not be sufficiently removed. For example, the process may include injecting supercritical carbon dioxide to maintain the pressure in the reactor at 80 to 400 bar, 90 to 400 bar, 100 to 400 bar, 110 to 400 bar, 120 to 400 bar, 130 to 400 bar, 140 to 400 bar, 150 to 400 bar, 150 to 390 bar, 150 to 380 bar, 150 to 370 bar, 150 to 360 bar, 150 to 350 bar, 160 to 350 bar, 170 to 350 bar, 180 to 350 bar, 190 to 350 bar, 200 to 350 bar, 210 to 350 bar, 220 to 350 bar, 230 to 350 bar, 240 to 350 bar, 250 to 350 bar, 260 to 350 bar, 270 to 350 bar, 280 to 350 bar, 290 to 350 bar, or 300 to 350 bar, but is not limited thereto.

In an embodiment, the injecting supercritical carbon dioxide into the reactor containing the biological tissue and ethanol may be performed for 1 to 24 hours, and, for example, may include maintaining the pressure in the reactor for 1 to 24 hours. For example, it may be 1 to 23 hours, 1 to 22 hours, 1 to 21 hours, 1 to 20 hours, 1 to 19 hours, 1 to 18 hours, 1 to 17 hours, 1 to 16 hours, 1 to 15 hours, 1 to 14 hours, 1 to 13 hours, or 1 to 12 hours, 2 to 12 hours, 2 to 11 hours, 2 to 10 hours, 2 to 9 hours, 2 to 8 hours, 2 to 7 hours, or 2 to 6 hours, but is not limited thereto. For example, the treating with the supercritical carbon dioxide may include maintaining the pressure in the reactor for 2 to 6 hours.

The decellularization method may further include a pretreatment prior to contacting the biological tissue with ethanol in the reactor. Through the pretreatment, contaminants, fat, and the like of the biological tissue may be removed.

The decellularization method may further include treating the mixture, which has undergone the process of injecting supercritical carbon dioxide into the reactor containing the biological tissue and ethanol, with a DNA-degrading enzyme, which serves to decompose DNA remaining in the mixture and thereby remove the immunogenicity of the mixture. In an embodiment, the DNA-degrading enzyme may be contained in distilled water at 0.01 to 1 µg/mL, and, for example, 0.01 to 0.9 µg/mL, 0.01 to 0.8 µg/mL, 0.01 to 0.7 µg/mL, 0.01 to 0.6 µg/mL, 0.01 to 0.5 µg/mL, 0.01 to 0.4 µg/mL, 0.01 to 0.3 µg/mL, 0.01 to 0.2 µg/mL, or 0.01 to 0.1 µg/mL, but is not limited thereto.

The decellularization method may further include a washing process of treating the mixture, which has undergone the process of treating with the DNA-degrading enzyme, with distilled water to remove residual contaminants...

In addition, another aspect provides a method of preparing a water-soluble dECM composition, including
treating a solution comprising a dECM powder with supercritical carbon dioxide, wherein the treating with the supercritical carbon dioxide is performed at 31 to 60 °C.

In addition, another aspect provides a water-soluble dECM composition prepared by the method of preparing a water-soluble dECM composition.

The terms or elements mentioned in the description of the method of preparing a water-soluble dECM composition and the water-soluble dECM composition prepared thereby that are the same as those already mentioned are as described above.

The water-soluble dECM composition may mean a composition including an aqueous solution containing a dECM, and may be a composition including a dECM dissolved by the method of dissolving dECM powder according to an embodiment.

### Advantageous Effects

According to the method of an aspect, in the process of dissolving a dECM through supercritical carbon dioxide treatment, the content and activity of bioactive substances in the dECM may be maintained, and low osmotic pressure may be maintained.

Therefore, the water-soluble dECM composition produced according to the method of an aspect, and the dECM material including the same, have low biotoxicity, not only possess excellent tissue regeneration ability, but also may be efficiently complexed with other substances, and thus may be usefully employed as cosmetic or medical materials.

### Description of Drawings

FIG. 1 is a result of visually confirming the freeze-dried dECM powder derived from pig skin tissue according to an embodiment.
FIG. 2 is a result of visually confirming the freeze-dried dECM powder derived from pig cardiac tissue according to an embodiment.
FIG. 3 is a result of confirming the dissolution of the freeze-dried dECM powder derived from pig skin tissue by injection of supercritical carbon dioxide according to an embodiment.
FIG. 4 is a result of confirming the dissolution of the freeze-dried dECM powder derived from pig skin tissue by injection of supercritical carbon dioxide according to an embodiment.
FIG. 5 is a result of confirming the dissolution of the freeze-dried dECM powder derived from pig cardiac tissue by injection of supercritical carbon dioxide according to an embodiment.
FIG. 6 is a result of comparing the pH of a composition according to the dissolution method of the freeze-dried dECM powder according to an embodiment.
FIG. 7 is a result of comparing the osmotic pressure of a composition according to the dissolution method of the freeze-dried dECM powder according to an embodiment.
FIG. 8 is a result of comparing the molecular weight of collagen in the dECM according to the dissolution method of the freeze-dried dECM powder according to an embodiment.
FIG. 9 is a result of comparing the poly dispersity index (PDI) of collagen in the dECM according to the dissolution method of the freeze-dried dECM powder according to an embodiment.

### Mode for Invention

Hereinafter, the present disclosure will be described in more detail through examples. However, these examples are provided for illustrative purposes only, and the scope of the present disclosure is not limited to these examples.

### Preparation Example 1. Preparation of dECM

### 1-1. Preparation of dECM Derived from Skin Tissue

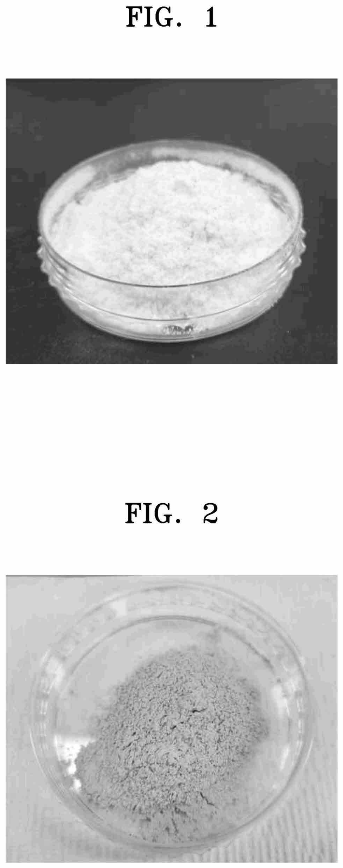

Pig skin tissue was prepared, exfoliated with a knife, and subjected to a pretreatment process to remove fat by stirring with 70 % ethanol at room temperature for 4 hours. Thereafter, 20 g of the skin tissue and 100 mL of 100 % ethanol were placed into a supercritical fluid apparatus reactor (Ilshin Autoclave, Korea), and the temperature was set to 35 °C. Thereafter, carbon dioxide was injected to adjust the pressure to 300 bar, and the reaction was carried out for 6 hours under the above temperature and pressure conditions. The tissue subjected to the above reaction was treated with a DNA-degrading enzyme solution (0.01 µg/mL), stirred for 24 hours, and then washed with distilled water. The washed tissue was freeze-dried and then pulverized using a freeze-miller. The result of visually confirming the dECM powder derived from pig skin tissue prepared in this manner is shown in FIG. 1.

### 1-2. Preparation of dECM Derived from Cardiac Tissue

Pig cardiac tissue was prepared, subjected to a pretreatment process of stirring in a 100 U/mL of heparin solution for 4 hours to remove blood, and then stirring in 70 % ethanol at room temperature for 4 hours to remove fat. Thereafter, 20 g of the cardiac tissue and 100 mL of 100 % ethanol were placed into a supercritical fluid apparatus reactor (Ilshin Autoclave, Korea), and the temperature was set to 35 °C. Afterwards, carbon dioxide was injected to adjust the pressure to 300 bar, and the reaction was performed for 6 hours under the above temperature and pressure conditions. The tissue subjected to the above reaction was treated with a DNA-degrading enzyme solution (0.01 µg/mL), stirred for 24 hours, and then washed with distilled water. The washed tissue was freeze-dried and then pulverized using a freeze-miller. The result of visually confirming the dECM powder derived from pig cardiac tissue prepared in this manner is shown in FIG. 2.

### Example 1. Dissolution of dECM Powder through Supercritical Carbon Dioxide Process

### 1-1. Dissolution of dECM Powder Derived from Skin Tissue

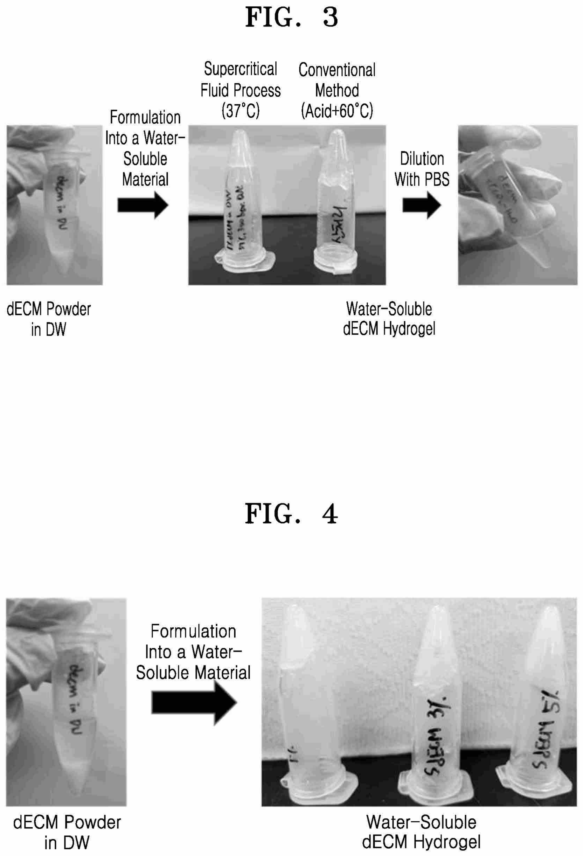

The dried dECM powder prepared as in Preparation Example 1-1 was mixed into distilled water at 1.0 wt% and placed into a supercritical fluid apparatus reactor (Ilshin Autoclave, Korea), and the temperature was set to 37 °C. Thereafter, carbon dioxide was injected to maintain the pressure in the reactor at 350 bar, and the reaction was carried out under such conditions for 12 hours. As a result, as shown in FIG. 3, it was confirmed that the dried powder of the dECM was sufficiently dissolved in the distilled water.

The dried dECM powder prepared as in Preparation Example 1-1 was mixed into distilled water at 1.0 wt%, 3.0 wt%, and 5.0 wt%, respectively, and placed into a supercritical fluid apparatus reactor (Ilshin Autoclave, Korea), and the temperature was set to 37 °C. Thereafter, carbon dioxide was injected to maintain the pressure in the reactor at 190 bar, and the reaction was carried out under such conditions for 12 hours. As a result, as shown in FIG. 4, it was confirmed that the dried powder of the dECM was sufficiently dissolved in the distilled water.

### 1-2. Preparation of Aqueous Solution of dECM Powder Derived from Cardiac Tissue

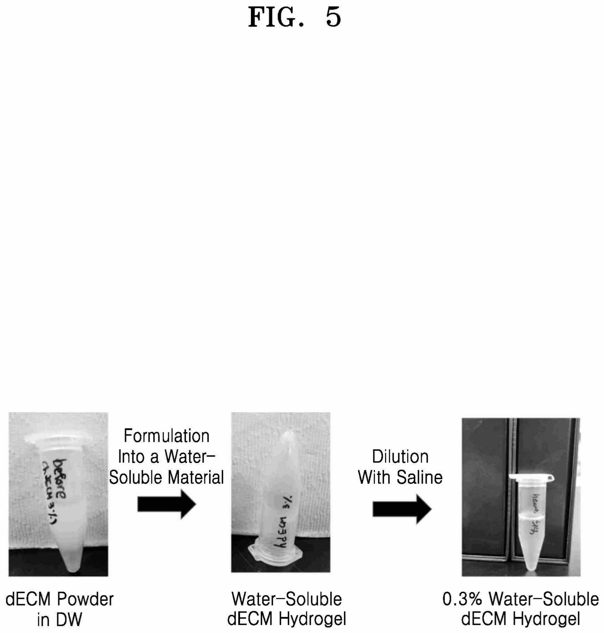

The dried dECM powder prepared as in Preparation Example 1-2 was mixed into distilled water at 3.0 wt% and placed into a supercritical fluid apparatus reactor (Ilshin Autoclave, Korea), and the temperature was set to 37 °C. Thereafter, carbon dioxide was injected to maintain the pressure in the reactor at 190 bar, and the reaction was carried out under such conditions for 12 hours. Thereafter, the hydrogel obtained through the above reaction was diluted tenfold with saline to prepare a 0.3% aqueous solution of the dECM. As a result, as shown in FIG. 5, it was confirmed that the dried powder of the dECM was sufficiently dissolved in the distilled water.

The method of dissolving the dried powder of the dECM through the supercritical carbon dioxide process of this Example used distilled water as a solvent, and was performed at a specific temperature below 60 °C. In addition, it did not include an additional dissolution method using an acidic or alkaline solvent. That is, through the supercritical carbon dioxide process as described above, it was confirmed that dissolution of the dECM powder is possible without acidic or alkaline solvents and without high-temperature conditions. These results indicate that the method of dissolving the dECM powder according to an embodiment maintains the content of effective bioactive substances in the dECM, prevents denaturation such as thermal decomposition, increases biological activity, exhibits low or no toxicity to biological tissue with excellent biocompatibility, and enables low osmotic pressure of the composition, thereby contributing to efficient compounding with other components.

### Experimental Example 1. Comparison of pH of dECM According to Dissolution Method (FIG. 3)

In this Experimental Example, in order to evaluate the pH of a dECM composition according to the dissolution method of an embodiment, it was compared with the pH of a dECM composition prepared by a conventional dissolution method.

Comparative Example 1 was prepared by dispersing the dried powder of the dECM prepared according to Preparation Example 1-1 at 1.0 wt% in 0.1 N HCl, and dissolving it at 60 °C for 1 hour. The pH thereof was compared with that of the dECM composition prepared according to Example 1-1.

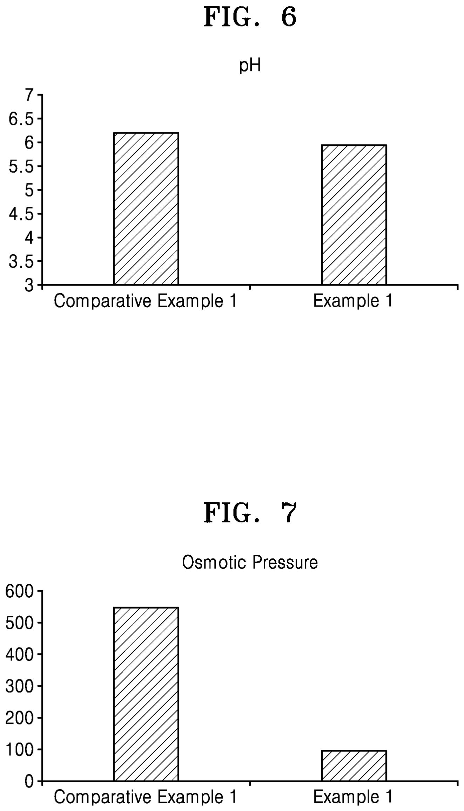

As a result, as shown in FIG. 6, it was confirmed that the dECM composition according to the dissolution method of an embodiment also maintained a pH of about neutral. These results indicate that the dissolution method according to an embodiment does not additionally require a process of adjusting the pH with an acidic or alkaline substance.

### Experimental Example 2. Comparison of Osmotic Pressure of dECM According to Dissolution Method

In order to compare the osmotic pressure of a dECM composition according to the dissolution method of the dECM powder, the osmotic pressure of the compositions according to Comparative Example 1 and Example 1-1 was measured using an osmometer (Osmomat 3000, Gonatec).

As a result, as shown in FIG. 7, it was confirmed that the dECM composition according to the dissolution method of an embodiment had significantly lower osmotic pressure compared to the comparative example. These results indicate that the water-soluble dECM composition according to the dissolution method of an embodiment is easy to compound with other substances and may serve as a material for effective composite cosmetic or medical materials.

### Experimental Example 3. Measurement of Molecular Weight and Distribution of Collagen in dECM According to Dissolution Method

In order to confirm the collagen content profile in a dECM composition according to the dissolution method of dECM powder, the following experiment was performed.

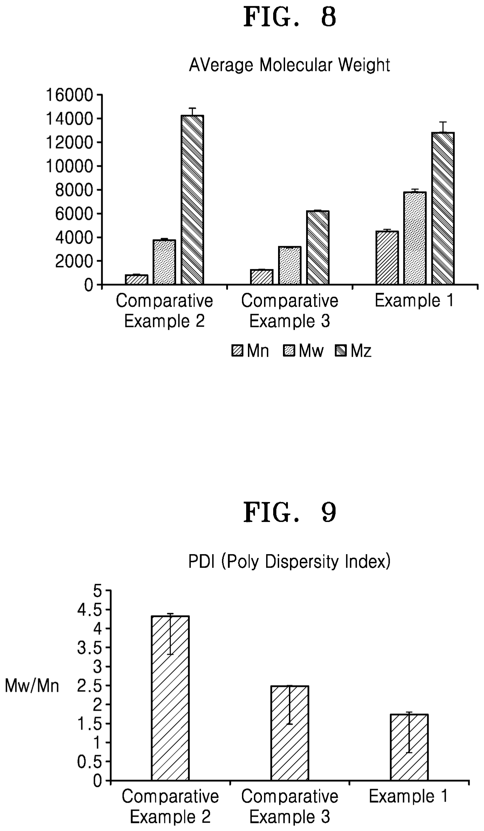

Comparative Example 2 was prepared by dispersing the dried powder of the dECM of Preparation Example 1-1 at 1 wt% in 0.1 N HCl containing pepsin and dissolving it at 4 °C for 48 hours. Comparative Example 3 was prepared by dispersing the dried powder of the dECM of Preparation Example 1-1 at 1.0 wt% in 0.1 N HCl containing pepsin and dissolving it at 60 °C for 1 hour. The molecular weight and molecular weight distribution of collagen in Comparative Examples 2 and 3 were compared with those of the composition according to Example 1-1. Through gel permeation chromatography (GPC) analysis, the number average molecular weight (Mn), weight average molecular weight (Mw), and Z-average molecular weight (Mz) of collagen in the decellularized materials prepared, respectively, were measured, and the results are shown in FIG. 8. In addition, based on the above results, the poly dispersity index (PDI, PDI = Mw/Mn) was calculated, and the results are shown in FIG. 9.

Through this, it was confirmed that, in the case of the dECM composition according to the dissolution method of an embodiment, collagen is contained not only while maintaining a higher molecular weight compared to the comparative examples (Comparative Examples 2 and 3), but also while maintaining a uniform molecular weight distribution. These results indicate that, in the case of the dECM according to the solubilization method of an embodiment, the biological activity is better maintained.

The foregoing description of the present disclosure is intended to be illustrative, and it will be understood by those of ordinary skill in the art to which the present disclosure pertains that various modifications may be easily made in other specific forms without changing the technical spirit or essential characteristics of the present disclosure. Therefore, the embodiments described above should be understood as illustrative in all respects and not as restrictive.

## Claims

1. A method of dissolving a decellularized extracellular matrix (dECM), comprising treating a solution comprising a dECM powder with supercritical carbon dioxide,
wherein the treating with the supercritical carbon dioxide is performed at 31 to 60 °C.

2. The method of claim 1, wherein the treating with the supercritical carbon dioxide is performed in a reactor, and carbon dioxide is injected to maintain a pressure in the reactor at 70 to 400 bar.

3. The method of claim 1, wherein the treating with the supercritical carbon dioxide is performed for 6 to 12 hours.

4. The method of claim 1, wherein the solution comprises 0.1 to 5 wt% of the dECM powder.

5. The method of claim 1, wherein the dECM is derived from any one selected from the group consisting of skin tissue, cardiac tissue, adipose tissue, corneal tissue, and cartilage tissue.

6. The method of claim 1, wherein the dECM is
prepared by a decellularization method comprising injecting supercritical carbon dioxide into a reactor containing biological tissue and ethanol to maintain a pressure in the reactor at 70 to 400 bar,
wherein the injecting supercritical carbon dioxide into the reactor containing biological tissue and ethanol is performed at 31 to 60 °C.

7. A method of preparing a water-soluble dECM composition, comprising treating a solution comprising a dECM powder with supercritical carbon dioxide,
wherein the treating with the supercritical carbon dioxide is performed at 31 to 60 °C.

8. A water-soluble dECM composition prepared by the method of claim 7.
